# EUROPEAN PATENT APPLICATION

(11) **EP 0 560 546 A1**
(43) Date of publication of application: **15.09.1993**
(21) Application number: 93301717.0
(22) Date of filing: 08.03.1993
(51) Int. Cl.: C07C 29/124, C07C 17/10, C07C 19/02, C07C 31/04

(54) **Production for preparaing alkanols and alkyl halides**

(30) Priority: 10.03.1992 US 848814
(71) Applicant: EXXON RESEARCH AND ENGINEERING COMPANY, Florham Park, New Jersey 07932-0390 (US)
(72) Inventor: Horvath, Istvan Tamas, New Hope, Pennsylvania 18938 (US); Millar, John Manuel, Pittstown, New Jersey 08867 (US); Cook, Raymond Arnold, Hampton, New Jersey 08827 (US)
(74) Representative: Fletcher Watts, Susan J.

(57) **Abstract**

An alkanol is produced from a monoalkyl halide by hydrolysis in the presence of a transition metal halide complex. The alkyl halide may be produced in situ from the corresponding alkane employing a system containing the said complex.

## Description

This invention relates to the production of alkanols from alkyl monohalides, which alkyl monohalides may be prepared in situ from alkanes.

Alkyl halides are known to those having ordinary skill in the art to have utility as a feedstock for more valuable commercial reactions. For example, methyl chloride and other alkyl halides have utility as an intermediate for production of alcohols, such as methanol, which itself is useful as an alternative, less environmentally damaging, fuel source. Additionally, methanol can be used as a feedstock for chemical reactions; for example, it can be used in reactions to yield gasoline or other hydrocarbons. More importantly, alcohol can be used as a transportation fuel source or as an additive to transportation fuels, particularly gasoline, to reduce hydrocarbon emissions and produce a more environmentally safe fuel.

In its broadest aspect, the invention provides a process for selectively producing an alkanol from a corresponding alkyl halide comprising hydrolysing the alkyl halide in the presence of a transition metal halide complex under hydrolysis conditions which are such that the total halogen/halide in the system does not decrease below that in the complex.

In accordance with a feature of the invention, the alkyl halide may be formed in situ from the corresponding alkane.

Thus, in accordance with another aspect of the invention, there is provided a process of selectively producing an alkyl monohalide and/or corresponding alkanol, which process comprises: forming a liquid phase containing water, at least one alkane, a transition metal halide complex and an added halogen source in addition to that contained in the transition metal halide complex; reacting the alkane, added halogen source and transition metal halide complex mixture at a temperature and for a sufficient time and in amounts that are effective to produce the corresponding alkyl monohalide and/or alkanol; and wherein, at least in the production of the alkanol, the added halogen source is not completely depleted.

The literature describes a number of processes for monohalogenating alkanes. However, unlike the processes described in the literature, applicants' process monohalogenate alkane(s) in a process that (i) uses a homogeneous liquid phase/transition metal complex system, (ii) uses no metal-bound halogen sources, (iii) operates in the presence of the transition metal halide complexes, (iv) shows no evidence of metallic platinum formation during the reaction until the added halogen source is totally depleted, and (v) is highly selective for monohalogenated products and their hydrolysis products.

The alkanes suitably may be methane, ethane or higher alkanes. The halogens are preferably chlorine, fluorine or bromine. The corresponding alcohol(s) may be produced during the reaction from the hydrolysis of the resulting alkyl halides or in a separate hydrolysis step.

The transition metal halide complex can be added to the system or may be produced in situ, from a compound consisting of a transition metal and a ligand capable of reacting to form the complex provided that the resulting complex is homogeneous with the system. Particularly useful transition metal halide complexes are those in which the transition metal is platinum, palladium, and nickel or mixtures thereof or, more preferably, platinum; and the halide is fluoride, chloride, bromide, or iodide or mixtures thereof, preferably chloride. The preferable combination of transition metal and halide is that of platinum and chloride; for example, as (PtCl₄)-² and (PtClₛ)-² together, or as (PtC1₄)-² alone. The cation portion of the complex may be a Group IA or IIA element, preferably H⁺, K⁺, or Na⁺. The particular complex may be prepared by methods known in the art or obtained from commercial sources. The complex should be homogeneous with the aqueous solution in the system. The term aqueous solution also includes solutions in, or containing, deuterium oxide.

The Periodic Table of Elements employed herein is that shown on page 789 of "The Condensed Chemical Dictionary", 10th Edition, Van Nostrand Reinhold Company, New York. The term "transition ele- ment/metal" is defined on page 1036 of said Dictionary.

The complex useful in the process of the present invention, and the aqueous solution also, may be supported in a solid hydrophilic support. In general, the support will be a porous solid material. For example, materials having a pore volume relative to solid weight of from about 0.1 to 1.5 cubic centimeters per gram, with a preferred range of from about 0.4 to 1.0 are especially useful supports. The macropore volume of the porous support should be at least 10% of the total pore volume. By macropore volume is meant pores having diameters greater than 100 Angstroms.

Specific examples of such materials useful as a support for the homogeneous liquid phase system in the practice of the process of the present invention are silica, clay, alumina, silica/alumina, acid treated clay and titania. Indeed, it is particularly preferred in the practice of the present invention to use acidic porous support materials such as silica/alumina, clay and, even more particularly, acid treated clay.

In a supported homogeneous liquid phase system, the transition metal complex used in the process of the present invention is dissolved in a supported aqueous acid phase; i.e., an aqueous acid phase that does not circulate or flow as a liquid, but is immobile and supported by the porous solid support. Typical supported aqueous acid liquid phase materials that may be used in the practice of the present invention include aqueous solutions ofHCI, HF, CH₃COOH, CF₃ COOH, H₃P0₄, H₂S0₄, CH₃SO₃H, CF₃SO₃H, BF₃ and mixtures thereof, preferably HCI, HF and mixtures thereof.

The volume of the supported aqueous phase will generally be a predetermined maximum amount that can be supported without causing the particles of the support to stick together, which amount may readily be determined by one ordinarily skilled in the art. The amount of aqueous phase should be less than that of the pore volume of the specific support employed. Indeed, it is preferred that the amount of aqueous phase will be about 10% less than the pore volume of the support. Thus, for example, about 1.3 cc of aqueous phase will be used with a support having a pore volume of 1.5 cc/gm.

Finally, in the homogeneous liquid phase system used in the process of the present invention, there is included a transition metal halide complex which is dissolved in the aqueous phase. The transition metals that may be employed herein include nickel, cobalt, rhodium, iridium, palladium, platinum, ruthenium, rhenium and mixtures thereof, but preferred is platinum.

In preparing a supported homogeneous liquid phase system, the transition metal complex is first dissolved in the aqueous phase, then the solution is impregnated into the porous support material by any appropriate means known to one skilled in the art, for example, by the incipient wetness technique.

The added halogen source used in the practice of the present invention should be additional to any halide contained in the transition metal halide complex having the transition metal halide. The source may be any Group VIIA element or compound, any Group VIIA-containing compound, and any other Group VIIA-containing species that exist(s) as an equilibrium product of the reaction of the Group VIlAelement, compound or Group VIIA-containing species in water in the presence of the complex and mixtures thereof. The added halogen source may be in elemental, molecular, ionic, free radical, other form or species, or mixtures thereof that are consistent with the chemical composition of the source. It may be introduced into the system in gaseous, liquid or other form that is or becomes soluble or dissolves in whole or in part in water, or it may be present in the system as an equilibrium product of the reactions involved in the particular system. The added halogen source is preferably a halogen, halide and hypohalite, or mixtures thereof, more preferably chlorine, a chloride and a hypochlorite; even more preferably, Cl₂ and HOCI, most preferably C1₂.

In the process of the present invention, monohalogenation of the alkanes is carried out under relatively mild conditions. Suitable monohalogenations may be carried out at a temperature range from about 20°C to about 315°C, preferably from about 20°C to about 200°C, more preferably from about 25° to about 150°C; and hydrolysis to the corresponding alcohols may be accomplished at temperatures from about 20°C to about 315°C, preferably from about 100°C to about 250°C, more preferably from about 100°C to about 150°C. The total pressure selected will vary based on the form in which the alkane and added halogen source are introduced into the system (e.g. liquid, gas), but generally for gaseous sources should be from about 1 atm to about 300 atm. Where, for example, the reaction is carried out using gaseous Cl₂ at about 20°C to about 25°C, the preferable pressure range is from about 1 to about 6 atm. Halogenation of alkanes to alkyl monohalides, according to the process of the present invention, may be carried out selectively, using the mole ratio of added halogen source to alkane of greater than or equal to about 1:1, preferably from about 1:1 1 to about 1:10, more preferably from about 1:1 to about 1:100.

If a significant amount of alcohol production is desired the reaction should be permitted to progress for a sufficient time to produce alcohol, but should not be allowed to proceed to the point that the added halogen source is depleted. The reactants should be used in effective amounts forthe production of the alkyl halide. Where the added halogen source is Cl₂ or HOCI and the complex containing the transition metal halide is Na₂PtCl₄/Na₂PtCl₆ or Na₂PtCI₄ alone, the process produces alkyl chlorides, particularly methyl chloride and ethyl chloride in high selectivity at pressures as low as about 5 atm, and at temperatures as low as about 100°C almost immediately. The reaction may be expected to proceed slowly, even at pressures as low as 1 atm.

In all cases, the pressure and temperature of the reaction and concentrations of reactants should be such that the flash point of the gaseous reactants is not exceeded. Due regard should be given to the corrosive nature of the particular Group VIIA reactants used. Particularly in the case of fluorination of alkanes, reactions should be performed in high dilution, preferably in the presence of an inert gas, to minimize handling problems. For processes known to those ordinarily skilled in the art for carrying out reactions using halogens, see e.g., F. Cotton and G. Wilkinson, Advanced Inorganic Chemistry. A Comprehensive Text, 4th ed., Part 2, Ch. 17, p.p. 542-576 "The Group VII Elements, Fluorine, Chlorine, Bromine, Iodine and Astatine".

The process of the present invention may be run in batch or may be operated continuously. The latter may be accomplished by removing on an ongoing basis the alkyl monohalides (or the alcohol, if the reaction is allowed to proceed under conditions which result in the hydrolysis to alcohol), recycling the complex containing the transition metal halide and regenerating the halogen source by oxidation of the halogen containing by-products of the reaction.

Reaction times for the process of the present invention will depend on the particular combination of reagents used, the sample size, and the type of process (batch or continuous), but should be sufficient to permit the synthesis of alkyl monohalides, and, if alcohol production is desired, the hydrolysis of the alkyl monohalides to alcohol. In order to maintain the selectivity of the reaction toward the production of alkyl monohalides, care should be taken such that the added halogen source is not depleted.

Subject to the foregoing limitations, reaction times generally needed to produce alcohol in quantities that are equal to or greater than those of, for example, alkyl chloride, are from about 15 minutes to about 16 hours at from about 100°C to about 150°C and from about 20 atm to about 80 atm, the more preferable time being from about 30 minutes to about 8 hours.

The selection of the particular reaction times, conditions and combination and concentrations of reagents will be readily apparent to one ordinarily skilled in the art given parameters established by the the teachings herein. General background concerning, for example, the conditions necessary for the chlorination of methane can be found in J. S. Sconce, Chlorine, Its Manufacture, Properties and Uses, R. Landau and S. Fox, Chapter 12, "Chlorinated Methanes", pp. 334 to 375. Other halogenation reactions may be carried out similarly by one ordinarily skilled in the art.

In order to maintain the high selectivityforalcohol production that is a characteristic of the present invention, it is important that the added halogen source not be totally depleted during the reaction. This may be accomplished by oxidizing the halide and oxyhalide by-products of the reaction to regenerate gaseous halogen and recycling them back into the system, or by adding additional halogen source to the reaction. Otherwise, at the point at which the added halogen source is totally depleted, the reaction may continue for a time and is identified by the formation of the metallic (zero valence) form of the transition metal portion of the complex (e.g., metallic platinum for the Na₂PtCl₄/Na₂PtCl₆ complex). It should be emphasized that in order to carry out chlorination, the transition metal (preferably platinum) complex is normally required in a concentration of from about 0.001 mole/liter to about 1 mole/liter, preferably from about 0.01 mole/liter to about 1 mole/liter, more preferably from about 0.1 mole/liter to about 0.5 mole/liter. However, in order to carry out the hydrolysis step, the transition metal (preferably platinum) complex is normally required in a concentration of from about 0.01 mole/liter to about 1 mole/liter.

The following examples are illustrative of aspects of the invention.

### Example 1

Commercially available chlorine, methane, Na₂ PtC1₄ and Na₂PtC1₆ were used without further purification. The reaction was performed at 125°C for 2 hours in an 8 ml sapphire high pressure nuclear magnetic resonance ("NMR") tube with 3 g of a D₂0 solution containing 1.2 mmol Na₂PtC1₆ and 0.16 mmol Na₂PtCI₄ at 72 psi Cl₂ and 392 psi ¹³CH₄. For an illustration of a sapphire NMR tube assembly, see I. T. Horvath and E. Ponce, "New Valve Design for High Pressure Sapphire Tubes for NMR Measurements", Review of Scientific Instruments, Vol. 62, No. 4, pp. 1104-1105 (1991). After 2 hours, the high pressure NMR spectrum showed the selective formation of methanol (approximately 40%, in the form of CH₃0D) as evidenced by the peak at about 49.5 ppm. NMRin- dicates the formation of trace amounts of CO₂, with a peak at about 125 ppm; CH₂(OD)₂ (approximately 2%), with a peak at about 85 ppm; CH₂Cl₂ (approximately 2%) with a peak at about 56 ppm; CH₃CI, (approximately 5%) with a peak at about 26 ppm; and unreacted methane (approximately 50%), with a peak at about -4 ppm. Formation of metallic platinum was not observed upon visual inspection of the transparent NMR tube under pressure. HCI that is formed as a byproduct during the formation of methanol may be treated with O2 to recoverthe chlorine. Methanol may be separated by distillation by processes known to one having ordinary skill in the art.

### Example 2

Commercially available methyl chloride was used without further purification. Hydrolysis was carried out in a sapphire high pressure NMR tube with 3g of a D₂0 solution containing 1.2 mmol Na₂PtCl₆and 0.16 mmole Na₂PtCI₄ at 50 psi CH₃CI and heated to 125°C for 1 hour. High pressure ¹³C NMR spectrum showed the selective formation of methanol (in the form of CH₃OD). The spectrum had the following characteristics: a peak at about 49.5 ppm, representing CH₃0D (approximately 49%); a peak at about 26 ppm for CH₃CI (approximately 50%).

## Claims

1. A process for selectively producing an alkanol from a corresponding alkyl halide comprising hydrolysing the alkyl halide in the presence of a transition metal halide complex under hydrolysis conditions which are such that the total halogen/halide in the system does not decrease below that in the complex.

2. The process as claimed in claim 1, wherein the alkyl halide is formed in situ from the corresponding alkane.

3. A process of selectively producing an alkyl monohalide and/or corresponding alkanol, which process comprises: forming a liquid phase containing water, at least one alkane, a transition metal halide complex and an added halogen source in addition to that contained in the transition metal halide complex; reacting the alkane, added halogen source and transition metal halide complex mixture at a temperature and for sufficient time and in amounts that are effective to produce the corresponding alkyl monohalide and/or alkanol; and wherein, at least in the production of the alkanol, the added halogen source is not completely depleted.

4. A process as claimed in claim 3, wherein the molar ratio of added halogen source to alkane is greater than or equal to 1:1.

5. A process as claimed in claim 3 or claim 4, wherein the added halogen source is selected from Group VIIAelement, a Group VIlAcompound and a Group VIIA-containing species, which exists as an equilibrium product in water, or the reaction of the Group VIIA element, compound or other Group VIIA-containing species and the transition metal halide complex.

6. A process as claimed in any one of claims 3 to 5, wherein the added halogen source is selected from a halogen, a halide, a hypohalite and mixtures thereof.

7. A process as claimed in any one of claims 3 to 6, wherein the added halogen source is a gas.

8. A process as claimed in any one of claims 3 to 7, wherein the liquid phase is supported on a solid, porous support.

9. A process as claimed in any preceding claim, wherein the alkyl monohalide is alkyl chloride.

10. A process as claimed in any preceding claim, wherein the transition metal halide contains platinum, palladium, nickel or mixtures thereof.

11. A process as claimed in any preceding claim, wherein the transition metal halide is selected from (PtCi₄)-² and (PtClₛ)-² together, or (PtCl₄)⁻² alone.

12. A process as claimed in claim 11, wherein the transition metal halide is (PtCl₄)⁻²and (PtCl₆)⁻²together.
